# EUROPEAN PATENT SPECIFICATION

(11) **EP 0 811 387 B1**
(45) Date of publication and mention of the grant of the patent: **05.03.2003**
(21) Application number: 96109173.3
(22) Date of filing: 07.06.1996
(51) Int. Cl.: A61L 15/18, A61L 15/46, A61F 13/15

(54) **Feminine hygiene absorbent products having a zeolite and silica odour control system**
Absorbierende Produkte für die Frauenhygiene mit Zeolith und Siliciumdioxid-Geruchskontrollesystem
Produits absorbants pour l'hygiène féminine contenant un système de contrôle des odeurs à base de zéolite et de silice

(43) Date of publication of application: 10.12.1997
(73) Proprietor: THE PROCTER & GAMBLE COMPANY, Cincinnati, Ohio 45202 (US)
(72) Inventor: Guarracino, Mario, 64028 Silvi Marina (IT); Gagliardini, Alessandro, 60035 Jesi (IT)
(74) Representative: Hirsch, Uwe Thomas

(56) References cited:
- EP-A- 0 389 015
- EP-A- 0 389 023
- EP-A- 0 510 619
- WO-A-81/01643
- DATABASE WPI Week 9014 Derwent Publications Ltd., London, GB; AN 90-104935 XP002020412 & JP-A-02 057 252 (OTA ) , 27 February 1990

## Description

The present invention relates to feminine hygiene absorbent products, in particular sanitary napkins and panty liners comprising compositions providing odour control benefits.

### Background of the Invention

Whilst the primary focus of feminine hygiene products remains the ability of these articles to absorb and retain fluids, another important area of development in this field is the control of odourous compounds contained within the absorbed fluids or their degradation products. There are a wide range of compounds which may be present in an feminine hygiene absorbent products during use which result in the formation of malodourous. These compounds include fatty acids, ammonia, amines, sulphur containing compounds and ketones and aldehydes. The malodours associated with the use of such products differ considerably with odours commonly related to the use of baby diapers which is not addressed by the present invention.

The art is replete with descriptions of various odour controlling agents for use in feminine hygiene products in order to address the problem of malodour formation. These agents can typically be classified according to the type of odour the agent is intended to combat. Odours may be classified as being acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid.

Neutral odour controlling agents have a pH of approximately 7. Examples of these types of compounds include activated carbons, clays, zeolites, silicas and starches. For example EPO 348 978 discloses an absorbent article comprising an odour control system wherein the neutral odour controlling particles are selected from carbon, clays, silicas, zeolites and molecular sieves. EPO 510 619 relates to absorbent article comprising odour control complex including a combination of at least 2 agents selected form a group which includes zeolites and silica gels. Similarly, WO 91/12029, WO 91/11977 and WO 91/12030 disclose the combination of zeolites and absorbent gelling materials.

Activated carbon or carbon black has been noted in the art as being particularly effective over a broad spectrum of odours. However, it is not favoured due to its black appearance, which is considered unacceptable by consumers. Hence a currently preferred odour control agent is zeolite. Although zeolites do not have a negative aesthetic profile, the main drawback is its lack of effective odour control over a broad range of odour types.

Hence, there still exists a need to provide an odour controlling agent or system which has an acceptable aesthetic profile such that it is light coloured and provides effective odour control over a wide range of malodourous compounds.

It has now been observed that this need may be addressed by the use of the combination of zeolite with silica to effectively combat a wide range of odours which may be present within an absorbent article. Surprisingly it has been observed that the combination of zeolite with silica results in a synergic effect such that the odour control performance of the combination of zeolite and silica is greater than the mere sum of the components.

An additional advantage of the present invention is that the compounds are all light in colour and thus are not noticeable within the absorbent article and are therefore acceptable from a consumer standpoint. Furthermore, the use of the combination zeolite and silica results in a reduction on costs of the odour control system.

None of the identified prior art has recognised the that this specific combination of zeolite and silica provides the above described benefits.

### Summary of the Invention

The present invention relates to a feminine hygiene absorbent product, being a sanitary napkin or a panty liner, comprising a liquid-pervious topsheet, a backsheet and an absorbent core, characterised in that said product comprises an odour control system comprising the combination of synthetic zeolite and silica at a ratio by weight of synthetic zeolite to silica from 1:5 to 5:1.

### Detailed Description of the Invention

The present invention relates to feminine hygiene absorbent products, namely sanitary napkins and panty liners. The absorbent products of the present invention comprise the essential features of a liquid pervious topsheet, a backsheet and an absorbent core intermediate the topsheet and the backsheet. The absorbent products further comprise as an essential component an odour control system.

### Odour control system

According to the present invention the absorbent product comprises as an essential feature an odour control system comprising a synthetic zeolite and silica, which is effective over a wide range of malodours.

### Zeolite odour control agent

The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula:

M_{2/n}O . Al₂O₃. ySiO₂ . wH₂O

where y is 2 or greater, n is the cation valence, and w is the water content in the voids of the zeolite.

Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

M_{x/n} [(AlO₂)ₓ (SiO₂)_{y}] . wH₂O

where n is the valence of cation M, w is the number of water molecules per unit cell, x and y are the total number of tetrahedra per unit cell, y/x usually having values of 1-5.

Zeolites may be naturally derived or synthetically manufactured. The synthetic zeolites are used herein. Suitable zeolites for use herein include zeolite A, zeolite P, zeolite Y, zeolite X, zeolite DAY, zeolite ZSM-5, and mixtures thereof. Most preferred are zeolite A or zeolite Y or mixtures thereof.

According to the present invention the zeolite is preferably hydrophobic. This is typically achieved by increasing the molar ratio of the SiO₂ to AlO₂ content such that the ratio or x to y is at least 1, preferably from 1 to 500, most preferably from 1 to 6.

The absorbent article preferably comprises from 40gm⁻² to 90gm⁻², more preferably from 55gm⁻² to 85gm⁻², most preferably from 60gm⁻² to 65gm⁻² of said zeolite.

### Silica odour control agent

According to the present invention the odour control system comprises as an essential component silica in combination with a synthetic zeolite. Silica i.e. silicon dioxide SiO₂ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that it contains at least 90%, preferably 95%, more preferably 99% silicon dioxide. Most preferably the silica is silica gel having a 100% silica content. Alternatively, the silica may be provided from other sources such as metal silicates including sodium silicate.

The absorbent product preferably comprises from 40gm⁻² to 100gm⁻², more preferably from 60gm⁻² to 90gm⁻², most preferably from 60gm⁻² to 65gm⁻² of silica based on 100% purity.

The silica (100%) and synthetic zeolite are present in the odour control system at a ratio by weight of from 1:5 to 5:1, more preferably from 3:1 to 1:3, most preferably from 1:1.

According to the present invention the weight of the odour control system which may be used in the absorbent product can be readily determined by the skilled person bearing in mind the absorbent article dimensions. For example the absorbent product may comprise from 0.5g to 5g, preferably from 1g to 3g, most preferably from 1.5g to 2.5g of said odour control system.

According to the present invention the odour control system may comprise additional optional components such as absorbent gelling materials, antimicrobial agents, activated carbon, perfuming ingredients, masking agents and chelants, all of which are known to the those skilled in the art.

The odour control system may be incorporated into the absorbent product by any of the methods disclosed in the art, for example layered on the core of the absorbent article or mixed within the fibres of the absorbent core. The odour control system is preferably incorporated between two layers of cellulose tissue. Optionally the system may be bonded between two cellulose tissue layers with, for example, a hot melt adhesive or any suitable bonding system.

More preferably the odour control system is incorporated in a layered structure in accordance with the disclosure of WO 94/01069 or Italian patent application number TO 93A 001028. TO 93A 001028 describes a layered structure substantially as described in WO 94/01069 with the exception that TO 93A 001028 comprises a much higher quantity of absorbent gelling material in the intermediate layer which is between the fibrous layers (120gm⁻²) that would be incorporated as an optional component in the present invention. The intermediate layer comprises in particular a polyethylene powder as thermoplastic material which is mixed with the premixed odour control system of the present invention. The mixture is then heated such that the polyethylene melts and glues the laminate layers and components together. The bridges which form the bond points between the fibrous layers involve particles of AGM as well as particles of thermoplastic material. (The absorbent capacity of the AGM is unaffected by bonding.) The adhesive lines are preferably also placed on the edges of the laminate to ensure that the edges of the laminate stick and any loose odour control material does not fall out of the laminate.

### Absorbent core

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent core may have any thickness depending on the end use envisioned.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### The topsheet

According to the present invention the absorbent article comprises as an essential component a liquid-pervious topsheet. The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as woven and non woven fabrics and films. In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

### Backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film.

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance. Further, the backsheet can permit vapours to escape from the absorbent structure, i.e. be breathable, while still preventing extrudates from passing through the backsheet. Also breathable backsheets comprising several layers, e.g. film plus non-woven structures, can be used.

### Examples:

The sanitary napkins used in the following examples were Always (Always is a registered Trade Mark) as sold by the Procter & Gamble Company. Each napkin was opened by cutting the wrap around the perforated coverstock at its bottom face approximately along a longitudinal edge of the release paper which covers the external adhesive layer. The side of the absorbent fibrous core is then-exposed by slightly shifting the water impermeable plastic bottom layer and subsequently, the fibrous core is split into two halves, each having approximately the same thickness, along a plane which is parallel to the plane of the napkin itself. The odour control system is homogeneously distributed between these two fibrous layers which are then joined together to reconstitute the absorbent core.

The water impermeable inner backsheet is then put back into its original position and the wrap around perforated coverstock is sealed along the cut by means of a e.g. a double sided adhesive tape.

Samples were produced using the method above, containing the odour control systems as described hereinbelow. A commercially available Always sanitary napkin without modification was used as a reference.

The zeolite used is zeolite A, Wessalith CS, available from Degussa AG. The silica used are Syloblanc 82 or Silica Gel 123, available from Grace GmbH.

| Product | Zeolite (g/napkin) | Silica (g/napkin) |
|---|---|---|
| Reference | 0 | 0 |
| 1 | 1.0 | 0 |
| 2 | 0 | 1.0 |
| 3 | 0.53 | 0.53 |

### Odour control test

### Principle of the test

in vivo sniff test consists of providing the test products to the users, wearing the products and returning them for analysis by expert graders that express their judgment about the (Un)pleasantness of the odour of the pad.

Each test comprises five separate stages:
1. Consignment of products
2. Wearing of products
3. Product return and preparation of test samples
4. Sniff test
5. Statistical analysis of the data

### Consignment of the product

Women are chosen who are known to have an odour control problem. Each of five women selected were given one product per test sample individually packed in an anonymous bag.

### Wearing of the products

Each woman wears the products in an alternating way. For example: if the first woman wears the product A, then product B and then the product C, the second woman wears the products in order B, C, A and so on. This is to ensure that the products are worn under the same conditions. The products are worn for as per habit and are frozen (at least at -20°C) immediately after removal.

### Product return and preparation of the test samples

The products are collected every day and are kept frozen (-60°C) until all the used products from the same women have been collected. The used pads are thawed to room temperature for 2/3 hours before testing. The products are then weighed to estimate the loading of menstrual fluid. Among the products, one reference pad which has not been worn is added. The used products are then placed into an aluminium tray covered with a perforated aluminium sheet.

### Sniff test

Sniff test session takes place in a large air-conditioned room with relatively rapid air turnover and is performed by at least six graders who have to sniff all the products of the same woman in each sniff test session. The grader may use any convenient sniffing strategy during this time, but is asked to be consistent throughout the test. During a test, graders sniff on the perforated aluminium sheet for approximately 5 seconds; the graders sniffs products at several seconds intervals from each them. In these conditions every sniffer evaluates the odour of each series of products using a (Un)pleasantness scale which ranges from -10 (highest level of unpleasantness) to 5 (most pleasant). With this procedure, each grader compares MU (Unpleasantness) in the test session. The relative MU odour values from different products are assigned numbers. For example, in a test session, a sample that is perceived to be twice as strong as another is assigned twice as large a number. One that is perceived to be one-tenth as strong as another is assigned a number one-tenth as large, etc. In each test session, zero is used to designate neutral hedonicity, and + and - numbers are assigned in ratio proportion to the relative pleasantness and unpleasantness of the odour.

The Unpleasantness values, for each sample, is obtained as a mean of at least 72 observations (six women, two products each, six graders).

### Statistical analysis of the data

The results collected from the test are then analysed by statistical analysis software (SAS). The data are processed to show statistically significant differences among untreated and treated products. The difference is shown in the tables by means of a letter near every mean value. Results with the same letter are not statistically significantly different. Duncan's multiple range test is used to form multiple comparisons.

### Results

Using the above method values of the (Un)pleasantness of the odour (MU) are obtained. Generally MU values are negative i.e. the higher the negativity the stronger the unpleasantness of the odour. The MU value gives an indication of the effectiveness of an odour control system.

| Product | Unpleasantness (MU) | % unpleasantness reduction |
|---|---|---|
| Reference | -3,3 B | 0% |
| 1 | -2.8 B | 15% |
| 2 | -2.9 B | 12% |
| 3 | -2.2 A | 33% |

As can be seen from the above results a 1g odour control system comprising both silica and zeolite provides significant improved odour control benefits in comparison to a 1g odour control system comprising only silica or only zeolite.

## Claims

1. A feminine hygiene absorbent product, being a sanitary napkin or a panty liner, comprising a liquid-pervious topsheet, a backsheet and an absorbent core, **characterised in that** said product comprises an odour control system comprising the combination of synthetic zeolite and silica at a ratio by weight of synthetic zeolite to silica from 1:5 to 5:1.

2. A feminine hygiene absorbent product according to claim 1, wherein said zeolite has a molar ratio of SiO₂ to AlO₂ of from at least 1.

3. A feminine hygiene absorbent product according to any one of the preceding claims, wherein said zeolite has a molar ratio of SiO₂ to AlO₂ of from 1 to 500.

4. A feminine hygiene absorbent product according to any one of the preceding claims, wherein said zeolite is selected from zeolite A, zeolite X, zeolite Y, zeolite P, zeolite DAY, zeolite ZSM-5 or mixtures thereof.

5. A feminine hygiene absorbent product according to any one of the previous claims, wherein said zeolite is zeolite A.

6. A feminine hygiene absorbent product according to any one of the preceding claims, wherein said product comprises from 0.5g to 5g of said odour control system.

7. A feminine hygiene absorbent product according to any one of the preceding claims, wherein said product comprises from 40gm⁻² to 90gm⁻² zeolite.

8. A feminine hygiene absorbent product according to any one of the preceding claims, wherein said product comprises from 40gm⁻² to 100gm⁻² silica.

## Patentansprüche

1. Absorbierendes Hygiene-Produkt fiir Frauen, das eine Monatsbinde oder eine Slip-Einlage ist, umfassend eine flüssigkeitsdurchlässige Decklage, eine Außenlage und einen absorbierenden Kern, **dadurch gekennzeichnet, dass** das Produkt umfasst ein Geruchs-Steuerungs-System mit der Kombination aus synthetischem Zeolith und Silika bei einem Gewichts-Verhältnis synthetischen Zeolith zu Silika von 1:5 bis 5:1.

2. Absorbierendes Hygiene-Produkt für Frauen nach Anspruch 1, wobei der Zeolith ein molares Verhältnis von SiO₂ zu AlO₂ von mindestens 1 aufweist.

3. Absorbierendes Hygiene-Produkt für Frauen gemäß einem der vorherigen Ansprüche, wobei der Zeolith ein molares Verhältnis von SiO₂ zu AlO₂ von 1 bis 500 aufweist.

4. Absorbierendes Hygiene-Produkt für Frauen gemäß einem der vorherigen Ansprüche, wobei der Zeolith gewählt ist aus Zeolith A, Zeolith X, Zeolith Y, Zeolith P, Zeolith DAY, Zeolith ZSM-5 oder Gemischen davon.

5. Absorbierendes Hygiene-Produkt für Frauen gemäß einem der vorherigen Ansprüche, wobei der Zeolith Zeolith A ist.

6. Absorbierendes Hygiene-Produkt für Frauen gemäß einem der vorherigen Ansprüche, wobei das Produkt 0,5g bis 5g des Geruchs-Steuerungs-Systems aufweist.

7. Absorbierendes Hygiene-Produkt für Frauen gemäß einem der vorherigen Ansprüche, wobei das Produkt 40gm⁻² bis 90gm⁻² Zeolith aufweist.

8. Absorbierendes Hygiene-Produkt für Frauen gemäß einem der vorherigen Ansprüche, wobei das Produkt 40gm⁻² bis 100gm⁻² Silika aufweist.

## Revendications

1. Produit absorbant pour l'hygiène féminine, une serviette hygiénique ou un protège-slip, comprenant une feuille de dessus perméable aux liquides, une feuille de fond et une âme absorbante, **caractérisé en ce que** ledit produit comprend un système agissant sur les odeurs, comprenant une combinaison de zéolite synthétique et de silice, en un rapport pondéral zéolite synthétique/silice allant de 1:5 à 5:1.

2. Produit absorbant pour l'hygiène féminine selon la revendication 1, dans lequel ladite zéolite a un rapport molaire SiO₂/AlO₂ d'au moins 1.

3. Produit absorbant pour l'hygiène féminine selon l'une quelconque des revendications précédentes, dans lequel ladite zéolite a un rapport molaire SiO₂/AlO₂ compris entre 1 et 500.

4. Produit absorbant pour l'hygiène féminine selon l'une quelconque des revendications précédentes, dans lequel ladite zéolite est choisie parmi la zéolite A, la zéolite X, la zéolite Y, la zéolite P, la zéolite DAY, la zéolite ZSM-5 ou leurs mélanges.

5. Produit absorbant pour l'hygiène féminine selon l'une quelconque des revendications précédentes, dans lequel ladite zéolite est une zéolite A.

6. Produit absorbant pour l'hygiène féminine selon l'une quelconque des revendications précédentes, dans lequel ledit produit comprend de 0,5 g à 5 g dudit système agissant sur les odeurs.

7. Produit absorbant pour l'hygiène féminine selon l'une quelconque des revendications précédentes, dans lequel ledit produit comprend de 40 g/m² à 90 g/m² de zéolite.

8. Produit absorbant pour l'hygiène féminine selon l'une quelconque des revendications précédentes, dans lequel ledit produit comprend de 40 g/m² à 100 g/m² de silice.
